(19) Europäisches Patentamt
European Patent Office
Office européen des brevets

(11) Publication number: 0 350 697
A2

(12) **EUROPEAN PATENT APPLICATION**

(21) Application number: 89111581.8

(22) Date of filing: 26.06.89

(51) Int. Cl.[4]: C07C 69/96 , C07C 68/00

(30) Priority: 11.07.88 US 217248

(43) Date of publication of application:
17.01.90 Bulletin 90/03

(84) Designated Contracting States:
DE FR GB IT NL

(71) Applicant: GENERAL ELECTRIC COMPANY
1 River Road
Schenectady New York 12305(US)

(72) Inventor: Chang, Tony Chin-Teh
4 Bayhill
Clifton Park New York 12065(US)

(74) Representative: Catherine, Alain
General Electric France Service de Propriété
Industrielle 18 Rue Horace Vernet
F-92136 Issy-Les-Moulineaux Cedex(FR)

(54) **Preparation of organic carbonates by oxidative carbonylation using palladium-manganese catalyst.**

(57) Organic hydroxy compounds, preferably aromatic, are converted to carbonates by reaction with carbon monoxide and oxygen under pressure in the presence of elemental or chemically combined palladium, divalent or trivalent manganese, at least one tetraalkylammonium halide and at least one quinone, aromatic diol reduction product thereof or mixture thereof. The preferred species are palladium(II) acetate, manganese(III) acetylacetonate, tetra-n-butylammonium bromide and 1,4-quinone or hydroquinone.

EP 0 350 697 A2

# PREPARATION OF ORGANIC CARBONATES BY OXIDATIVE CARBONYLATION USING PALLADIUM-MANGANESE CATALYST

This invention relates to the oxidative carbonylation of organic hydroxy compounds, and more particularly to the preparation of organic carbonates.

Organic carbonates are useful reagents for many purposes. In particular, aryl carbonates are either polycarbonates or may be converted thereto by transesterification with a bisphenol or the like. This method of polycarbonate preparation is of interest because it does not require the use of phosgene, a toxic and irritating gas which is ordinarily employed in stoichiometric excess for the preparation of such polycarbonates.

Various catalytic methods for the reaction of organic hydroxy compounds with carbon monoxide and oxygen to form carbonates are known. Typical methods of this type are described, for example, in U.S. Patents 4,201,721, 4,349,485 and 4,361,519. They all involve the use of a noble metal catalyst, typically elemental or chemically combined palladium, and for the most part require basic conditions. Also required as a practical matter in most instances, particularly for the formation of aromatic carbonates, are such materials as manganese or cobalt co-catalysts, drying agents such as molecular sieves, and phase transfer catalysts or solvents such as methylene chloride. Certain of these required materials, especially the bases and drying agents, interact with the catalyst and co-catalyst materials and convert them to forms not adaptable to convenient regeneration and recycle. In addition, these procedures often require very long reaction times.

U.S. Patent 4,281,174 describes the reaction of alcohols with carbon monoxide and oxygen in the presence of a palladium catalyst, a quinone and a "redox agent" which may be a manganese or cobalt compound. The palladium catalyst is a complex containing ligands which may be amines, phosphines, arsines, stibines or halide or pseudohalide salts. The products, however, are not carbonates but dialkyl oxalates.

The present invention provides a method for preparing organic carbonates which does not require the presence of bases, drying agents or solvents, making catalyst recycle more practical. It is adaptable to produce carbonates in respectable yield, by a relatively simple procedure, and often in a relatively short time.

Accordingly, the invention is a method for preparing an organic carbonate which comprises contacting an organic hydroxy compound with carbon monoxide and oxygen at a temperature in the range of about 70-150° C and partial pressures of carbon monoxide and oxygen of at least about 30 and about 2 atmospheres, respectively, in the presence of:

(A) elemental, catalytically active palladium or chemically combined palladium in the amount of about 1 gram-atom per 800-10,000 moles of organic hydroxy compound,

(B) divalent or trivalent manganese in the amount of about 0.5-5.0 gram-atoms per gram-atom of palladium,

(C) at least one tetraalkylammonium halide in the amount of about 10-100 moles per gram-atom of palladium, and

(D) at least one quinone, aromatic diol reduction product thereof, or mixture thereof in the amount of about 20-50 moles per gram-atom of palladium.

The reactants employed in the method of this invention are an organic hydroxy compound, carbon monoxide and oxygen which may be provided in the form of air. The organic hydroxy compound may be an aliphatic, alicyclic or aromatic mono- or polyhydroxy compound such as methanol, ethanol, cyclohexanol, phenol, cresol, xylenol, ethylene glycol, propylene glycol, resorcinol, hydroquinone or bisphenol A. Aromatic hydroxy compounds and especially monohydroxy compounds are preferred, with phenol being most preferred.

According to the invention, the oxidative carbonylation reaction takes place in the presence of various chemical species serving as catalysts, redox agents and the like. Component A is palladium in elemental, catalytically active form or chemically combined form. Thus, palladium black or elemental palladium deposited on carbon are suitable, as well as palladium compounds such as halides, nitrates, carboxylates and complexes involving such compounds as carbon monoxide, amines, phosphines or olefins. Preferred in most instances are palladium(II) salts of organic acids, most often carboxylates with $C_{2-6}$ aliphatic acids. Palladium(II) acetate is most preferred.

Component B is chemically combined manganese, which may be divalent or trivalent. Again, any manganese compound within this class may be used, including simple salts such as halides and carboxylates and complexes with amines, diketones, carbon monoxide and the like. The manganese chelates with diketones of the formula

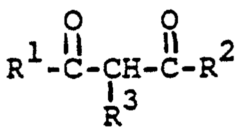 ,

wherein each of $R^1$ and $R^2$ is a primary or secondary alkyl radical containing about 1-5 carbon atoms and $R^3$ is hydrogen or a primary or secondary alkyl radical containing about 1-5 carbon atoms, are preferred, with manganese(III) acetylacetonate being most preferred.

Component C, the tetraalkylammonium halide, is critical for the formation of organic carbonates pursuant to the invention. The chlorides and bromides and especially the bromide are preferred, and the most useful alkyl groups are primary and secondary alkyl groups containing about 1-8 carbon atoms. Most preferred is tetra-n-butylammonium bromide.

Component D is at least one quinone, aromatic diol formed by reduction of said quinone, or mixture thereof. 1,4-Quinone and hydroquinone are preferred. Other suitable compounds are 1,2-quinone and catechol, and 9,10-anthraquinone and 9,10-dihydroxyanthracene.

According to the invention, contact between the organic hydroxy compound, carbon monoxide, oxygen and other components is effected under high pressure and at a temperature in the range of about 70-150 °C, preferably about 80-130 °C. The partial pressures of carbon monoxide and oxygen are at least about 30 and at least about 2 atmospheres, respectively, with preferred partial pressures being in the range of about 40-60 and about 2-10 atmospheres, respectively.

The proportions of components A, B, C and D are subject to wide variation, with certain preferences for the preparation of organic carbonate in maximum yield. Thus, component A is present in the amount of about 1 gram-atom of palladium per 800-10,000 and preferably 6000-10,000 moles of organic hydroxy compound. Proportions of other constituents are in terms of gram-atom of palladium and are, respectively, about 0.5-5.0 and preferably about 0.5-1.5 gram-atoms of manganese, about 10-100 and preferably about 50-80 moles of tetraalkylammonium halide, and about 20-50 and preferably about 25-40 moles of quinone and/or reduction product thereof.

It is within the scope of the invention to employ solvents in the reaction. Suitable solvents include aliphatic, alicyclic and aromatic hydrocarbons such as hexane, heptane, cyclohexane, toluene and decalin; halogenated hydrocarbons such as methylene chloride, chloroform and chlorobenzene; ethers such as diphenyl ether and dioxane, and esters such as ethyl acetate and methyl formate. Also contemplated is the use of drying agents such as activated alumina, calcium sulfate, calcium chloride and molecular sieves. However, under most conditions the use of solvents and drying agents offers no advantage and is therefore not preferred.

Recovery of the organic carbonate may be effected by conventional operations including, for example, distillation. The palladium may also be recovered, for example, by treatment with nitric acid and reconversion to a carboxylate or other compound particularly useful as component A.

The invention is illustrated by the following examples.

Example 1

A 300-ml. autoclave was charged with 75 grams (800 mmol.) of phenol, 23 mg. (0.1 mmol.) of palladium(II) acetate, 35 mg. (0.1 mmol.) of manganese(III) acetylacetonate, 1.93 grams (6 mmol.) of tetrabutylammonium bromide and 330 mg. (3 mmol.) of hydroquinone. The autoclave was sealed, pressurized with 54.9 atmospheres of carbon monoxide and 4.8 atmospheres of oxygen, heated at 120 °C for 1 hour, cooled and vented. Analysis by gas chromatography showed the presence of diphenyl carbonate in an amount corresponding to a phenol conversion of 7%.

Example 2

The procedure of Example 1 was repeated, except that the partial pressures of carbon monoxide and oxygen were 47.6 and 4.1 atmospheres, respectively, the reaction temperature was 90 °C and the reaction time was 7 hours. The product yield corresponded to 7.3% conversion of phenol.

Example 3

The procedure of Example 1 is repeated, substituting quinone on a equimolar basis for the hydroquinone. Similar results are obtained.

Example 4

The procedure of Example 1 is repeated, replacing the palladium(II) acetate with an equivalent amount of 10% palladium on carbon. Similar results are obtained.

Example 5

The procedure of Example 1 is repeated, substituting bis(diphenylphosphino)methane palladium(II) chloride for the palladium(II) acetate. Similar results are obtained.

Example 6

The procedure of Example 1 is repeated, with the addition of 6 grams of 3A molecular sieves. Similar results are obtained.

Example 7

The procedure of Example 1 is repeated, replacing the oxygen with air at 13.6 atmospheres (oxygen partial pressure 2.7 atmospheres). Similar results are obtained.

## Claims

1. A method for preparing an organic carbonate which comprises contacting an organic hydroxy compound with carbon monoxide and oxygen at a temperature in the range of about 70-150° C and partial pressures of carbon monoxide and oxygen of at least about 30 and about 2 atmospheres, respectively, in the presence of:

(A) elemental, catalytically active palladium or chemically combined palladium in the amount of about 1 gram-atom per 800-10,000 moles of organic hydroxy compound,

(B) divalent or trivalent manganese in the amount of about 0.5-5.0 gram-atoms per gram-atom of palladium,

(C) at least one tetraalkylammonium halide in the amount of about 10-100 moles per gram-atom of palladium, and

(D) at least one quinone, aromatic diol reduction product thereof, or mixture thereof in the amount of about 20-50 moles per gram-atom of palladium.

2. A method according to claim 1 wherein component A is a palladium(II) salt of an organic acid.

3. A method according to claim 2 wherein reagent A is palladium(II) acetate.

4. A method according to claim 2 wherein component B is a manganese chelate with a diketone of the formula

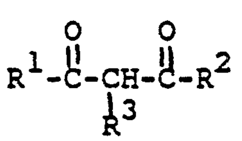

,

wherein each of $R^1$ and $R^2$ is a primary or secondary alkyl radical containing about 1-5 carbon atoms and $R^3$ is hydrogen or a primary or secondary alkyl radical containing about 1-5 carbon atoms.

5. A method according to claim 4 wherein reagent B is manganese(III) acetylacetonate.

6. A method according to claim 4 wherein component C is a tetraalkylammonium bromide in which the alkyl groups are primary or secondary alkyl groups containing about 1-8 carbon atoms.

7. A method according to claim 6 wherein component C is tetra-n-butylammonium bromide.

8. A method according to claim 6 wherein component D is at least one of 1,4-quinone and hydroquinone.

9. A method according to claim 8 wherein oxygen is provided in the form of air.

10. A method according to claim 8 wherein the organic hydroxy compound is phenol.

11. A method according to claim 9 wherein the reaction temperature is within the range of about 80-130° C.

12. A method according to claim 11 wherein component A is present in the amount of about 1 gram-atom of palladium per 6000-10,000 moles of organic hydroxy compound, component B in the amount of about 0.5-1.5 gram-atoms of manganese per gram-atom of palladium, component C in the amount of about 50-80 moles per gram-atom of platinum, and component D in the amount of about 25-40 moles per gram-atom of palladium.

13. A method according to claim 12 wherein the partial pressures of carbon monoxide and oxygen are in the range of about 40-60 and about 2-10 atmospheres, respectively.

14. A method according to claim 13 wherein the organic hydroxy compound is phenol.

15. A method according to claim 14 wherein reagent A is palladium(II) acetate, reagent B is manganese(III) acetylacetonate and reagent C is tetra-n-butylammonium bromide.

16. A method according to claim 15 wherein the oxygen is provided in the form of air.